# DEMANDE DE BREVET EUROPEEN

(11) **EP 3 012 213 A1**
(43) Date de publication de la demande: **27.04.2016**
(21) Numéro de dépôt: 15190552.8
(22) Date de dépôt: 20.10.2015
(51) Int. Cl.: B65D 83/30, A24F 47/00, A61M 15/06, B65D 83/14, A61M 11/04

(54) **DISPOSITIF DE REMPLISSAGE DE CIGARETTE ÉLECTRONIQUE**

(30) Priorité: 23.10.2014 BE 201405026
(71) Demandeur: Serenity SA, 7180 Seneffe (BE)
(72) Inventeur: RACHIDI, Saïd, B-7022 HYON (BE); MAY, Bronislav Henric, B-3090 OVERIJSE (BE)
(74) Mandataire: Pronovem

(57) **Abrégé**

La présente invention divulgue un dispositif de remplissage d'une cigarette électronique comprenant :
- un flacon (4) pressurisé contenant un liquide pour cigarette électronique;
- une valve de dosage (2) connecté à un tube (6) plongeant dans ledit liquide;
- un tuyau d'injection (5) connecté à la sortie de la valve de dosage (2).

## Description

### Objet de l'invention

La présente invention se rapporte à un dispositif de remplissage d'une cigarette électronique, et à une cigarette électronique adaptée à être remplie au moyen de ce dispositif.

### Etat de la technique

La cigarette électronique rencontre actuellement un succès grandissant par rapport à la cigarette traditionnelle. En effet, cette dernière comporte des risques de santé élevés liés aux produits de combustion inhalés par le fumeur. De plus en plus de fumeurs cherchent donc à arrêter de fumer, mais il est démontré qu'une dépendance s'installe très rapidement chez le fumeur et que le l'arrêt est souvent difficile. La cigarette électronique représente aujourd'hui un moyen commode de substitut à la cigarette traditionnelle.

Afin de maintenir les habitudes gestuelles du fumeur le temps du sevrage, les cigarettes électroniques présentent généralement une forme allongée, proche de celle des produits habituels du tabac (cigarettes, cigare ou pipe). De ce fait, le liquide vaporisé pour simuler la fumée doit être introduit dans un orifice de petite taille à l'extrémité supérieure d'un réservoir de petit diamètre. En outre, ce réservoir comprend en son centre une tubulure par laquelle l'air et le liquide vaporisé sont inhalés. Or, il est important, pour éviter d'ingérer directement le liquide, que cette tubulure concentrique ne reçoive aucune contamination directe du liquide à vaporiser. De ce fait, le remplissage à partir des conditionnements actuellement sur le marché est délicat et demande une dextérité importante.

Par ailleurs, les liquides à vaporiser comportent généralement d'importantes quantités de composés volatiles, parfois sensibles à l'oxydation et/ou à la lumière.

### But de l'invention

La présente invention vise à proposer un conditionnement permettant un remplissage aisé.

Selon des modes préférés de l'invention, le conditionnement de l'invention permet aussi une durée de conservation prolongée.

### Résumé de l'invention

La présente invention se rapporte à dispositif de remplissage d'une cigarette électronique comprenant :
a) un flacon pressurisé contenant un liquide pour cigarette électronique;
b) une valve de dosage connecté à un tube plongeant dans ledit liquide;
c) un tuyau d'injection (5) connecté à la sortie de la valve de dosage.

Selon des modes de réalisation préférés de l'invention, le dispositif de remplissage comprend une ou une combinaison adéquate de plusieurs des caractéristiques suivantes :
- le flacon comprend au moins une couche d'un matériau barrière au gaz sélectionné parmi le groupe consistant en métaux, métalloïdes, oxydes métalliques ou de métalloïdes, EVOH, polyamide, polyester et polymère à cristaux liquide ;
- le flacon est essentiellement métallique, préférablement recouvert d'un revêtement de nature différente, de préférence un polymère, destiné à protéger l'intégrité du contenant et/ou du contenu;
- Le flacon est en verre ;
- le dispositif comprend un gaz propulseur inerte ;
- la valve de dosage comprend des moyens permettant de dispenser une quantité fixe, prédéfinie de liquide pour cigarette électronique ;
- le tuyau d'injection comprend des moyens de connexion étanches permettant de se connecter, en utilisation, à des moyen correspondants sur le réservoir d'une cigarette électronique, lesdits moyens de connexion comprennent de préférence un luer mâle ou femelle, avantageusement le luer est de type luer lock.

Un second aspect de l'invention concerne une cigarette électronique comprenant un réservoir de liquide à vaporiser, ledit réservoir comprenant une valve unidirectionnelle de remplissage, ladite valve permettant le remplissage du réservoir et empêchant le liquide de s'écouler hors du réservoir, et la valve comprenant des moyens de connexion étanches au tuyau d'injection du dispositif de remplissage selon l'une quelconque des revendications précédentes.

Selon des modes de réalisation préférés de l'invention, la cigarette électronique comprend une ou une combinaison adéquate de plusieurs des caractéristiques suivantes :
- les moyens de connexion étanches comprennent un connecteur de type Luer femelle ou mâle ;
- le connecteur de type Luer est du type Luer Lock ;
- la valve unidirectionnelle comprend un ressort poussant une pièce mobile de fermeture contre le culot de la valve, la pression appliquée par le ressort entre la pièce mobile de fermeture et le culot assurant l'étanchéité du réservoir de la cigarette électronique.

### Description des figures

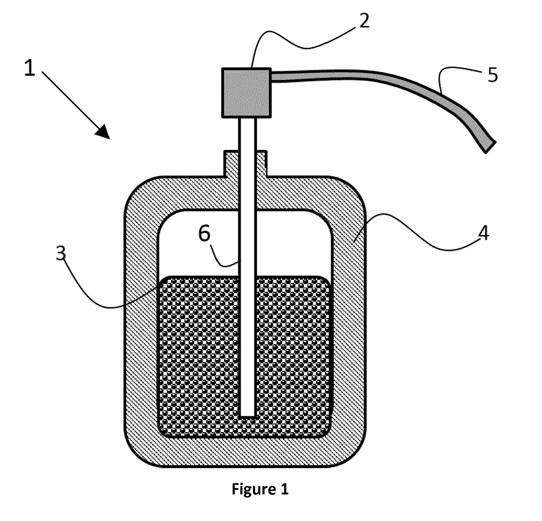

La figure 1 représente une vue en coupe d'un exemple de réalisation d'un dispositif de remplissage selon l'invention.

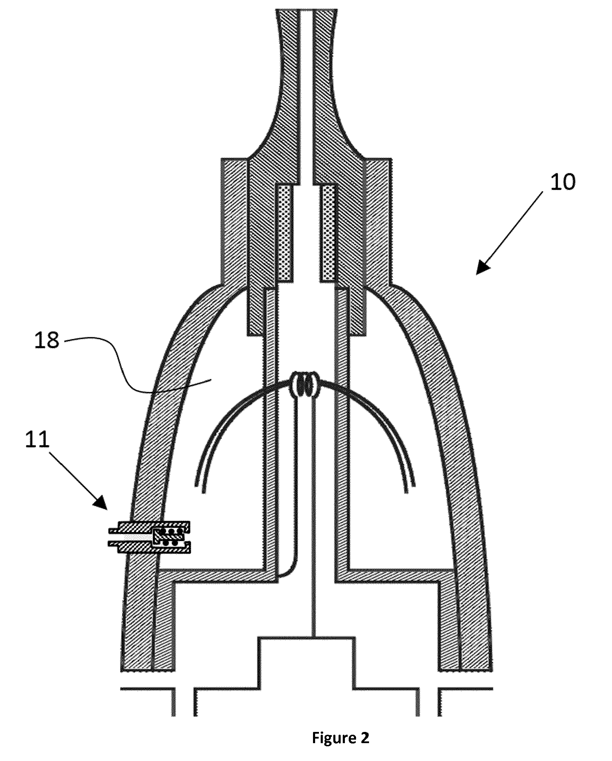

La figure 2 représente une vue en coupe d'un exemple de corps d'une cigarette électronique selon l'invention.

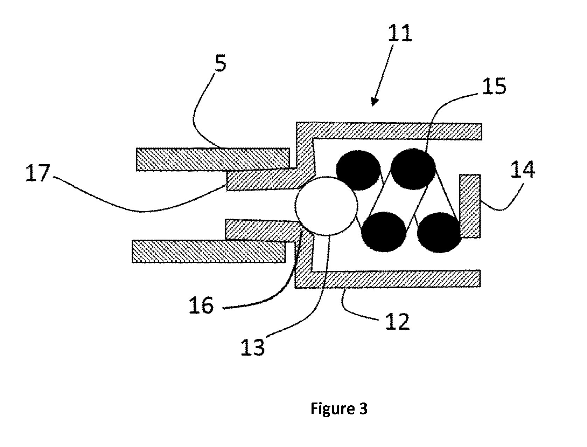

La figure 3 représente un exemple de valve unidirectionnelle pour cigarette électronique selon l'invention.

### Description détaillée de l'invention

Le dispositif de remplissage d'une cigarette électronique selon l'invention comprend un flacon 4 pressurisé contenant le liquide 3 pour cigarette électronique. La pressurisation du flacon 4 permet une injection aisée du liquide dans le réservoir de la cigarette, en particulier, sans qu'il soit nécessaire d'actionner une pompe ou de pousser de façon importante sur le corps du flacon, tel que dans les flacons de l'art antérieur.

Une valve de dosage 2 connecté à un tube 6 plongeant dans le liquide permet d'extraire celui-ci, par exemple en exerçant une pression sur la valve permettant l'ouverture de celle-ci. Cette valve est donc avantageusement du type de celles utilisée pour les bombes pressurisées de peinture, de parfum ou de crème fraîche.

Un tuyau d'injection 5 est connecté à la sortie de la valve de dosage 2. Ce tuyau d'injection est de préférence long et souple de façon à pouvoir être introduit dans l'orifice supérieur du réservoir 18 de la cigarette électronique sans difficulté.

Avantageusement, le dispositif de l'invention sert aussi d'emballage primaire du liquide à vaporiser pendant toute sa durée de conservation.

Le flacon 4 comprend avantageusement au moins une couche d'un matériau barrière au gaz, afin d'assurer une bonne conservation des arômes et des composés volatiles compris dans le liquide à vaporiser. Ce matériau barrière est avantageusement sélectionné parmi le groupe consistant en métaux, métalloïdes, oxydes métalliques ou de métalloide, EVOH, polyamide, polyester et polymère à cristaux liquide.

Avantageusement, le flacon est essentiellement constitué d'un métal ou de verre. Dans le cas d'un récipient métallique, il est préférablement recouvert d'un revêtement de nature différente, de préférence un polymère, destiné à protéger l'intégrité du contenant et/ou du contenu.

Afin de réduire l'oxydation des composés du liquide, la pressurisation est obtenue au moyen d'un gaz propulseur inerte tel que de l'azote, ou un gaz rare ou tout gaz compatible avec l'application.

Un second aspect de l'invention concerne une cigarette électronique 10 comprenant une valve 11 unidirectionnelle de remplissage. Cette valve unidirectionnelle est adaptée à former par pression ou par vissage une connexion étanche avec l'extrémité libre du tuyau de remplissage 5.

Avantageusement, le tuyau de remplissage est un tuyau court et rigide dont l'extrémité libre présente un profil du type luer mâle, tandis que l'entrée de la valve unidirectionnelle présente un profil de type luer femelle (non représenté). Dans ce cas, le remplissage sera effectué en pressant l'extrémité luer mâle du dispositif de remplissage 1 dans le luer femelle de la cigarette électronique. Avantageusement, la pression nécessaire à l'ouverture de la valve de dosage 2 sera supérieure à la pression nécessaire à l'étanchéité de l'assemblage de type luer.

De façon alternative, c'est le connecteur sur la cigarette électronique qui présente un profil conique de type luer mâle 17, tandis que l'extrémité libre du tuyau de remplissage 5 présente un diamètre très légèrement inférieur à l'extrémité du luer mâle 17, de façon à permettre un assemblage du tuyau par forçage et déformation élastique de l'extrémité du tuyau. Dans ce cas, l'extrémité du tuyau de remplissage est de préférence souple, voir caoutchoutique et présente avantageusement un module élastique inférieur à 200MPa, de préférence inférieur à 100MPa.

Afin de rendre le dispositif de remplissage de l'invention compatible avec tous types de cigarettes électroniques, le tuyau de remplissage 5 comprend au moins deux pièces, un tuyau court et rigide actionnant par pression la valve de dosage 2, et un tuyau souple pouvant être introduit directement dans le réservoir d'une cigarette électronique ou être connecté à un luer mâle connecté à une valve unidirectionnelle communiquant avec le réservoir d'une cigarette électronique. Ces deux pièces peuvent être jointes par un connecteur rigide servant de bouton poussoir pour actionner la valve unidirectionnelle. Dans ce cas, le tuyau de remplissage 5 sera adapté à être connecté de façon directe à un connecteur de type luer femelle selon le procédé de remplissage décrit ci-avant.

Les profiles luer sont défini dans la norme ISO 594-1. Ce sont des assemblages coniques à 6 % selon la norme. Par profile de type luer, on entend des assembles coniques similaires aux luer présentant des angles de cône compris entre 3 et 10°. Il est avantageux d'utiliser les dimensions des luers de la norme, ce qui permet éventuellement d'utiliser des seringues standards du type de celles utilisées dans le secteur médical pour remplir la cigarette électronique 10 de l'invention.

Afin de s'assurer de l'étanchéité de la connexion, les luers utilisés seront de préférence du type luer-lock.

la valve de remplissage unidirectionnelle (11) permet le remplissage du réservoir 18 et empêche le liquide de s'écouler hors du réservoir. Par exemple, la valve unidirectionnelle comprend un ressort 15 poussant une pièce mobile de fermeture 13 contre le culot 16 de la valve 11, la pression assurée par le ressort 15 entre la pièce mobile de fermeture 13 et le culot 16 assurant l'étanchéité du réservoir de la cigarette électronique 10.

## Revendications

1. Dispositif de remplissage d'une cigarette électronique comprenant :
- un flacon (4) pressurisé contenant un liquide pour cigarette électronique;
- une valve de dosage (2) connecté à un tube (6) plongeant dans ledit liquide;
- un tuyau d'injection (5) connecté à la sortie de la valve de dosage (2).

2. Dispositif de remplissage selon la revendication 1 dans lequel le flacon (4) comprend au moins une couche d'un matériau barrière au gaz sélectionné parmi le groupe consistant en métaux, métalloïdes, oxydes métalliques ou de métalloides, EVOH, polyamide, polyester et polymère à cristaux liquide.

3. Dispositif de remplissage selon l'une quelconque des revendications précédentes dans lequel le flacon est essentiellement métallique.

4. Dispositif de remplissage selon l'une quelconque des revendications précédentes dans lequel le flacon est en verre.

5. Dispositif de remplissage selon l'une quelconque des revendications précédentes comprenant un gaz propulseur inerte.

6. Dispositif de remplissage selon l'une quelconque des revendications précédentes dans lequel la valve de dosage comprend des moyens permettant de dispenser une quantité fixe, prédéfinie de liquide pour cigarette électronique.

7. Dispositif de remplissage selon l'une quelconque des revendications précédentes dans lequel le tuyau d'injection comprend des moyens de connexion étanches permettant de se connecter, en utilisation, à des moyens correspondants sur le réservoir d'une cigarette électronique.

8. Dispositif de remplissage selon la revendication 6 dans lequel lesdits moyens de connexion comprennent une connextion de type luer mâle ou femelle.

9. Dispositif de remplissage selon la revendication 7 dans lequel le connecteur de type luer est du type luer lock.

10. Cigarette électronique (10) comprenant un réservoir (18) de liquide à vaporiser, ledit réservoir comprenant une valve (11) unidirectionnelle de remplissage, ladite valve (11) permettant le remplissage du réservoir (18) et empêchant le liquide de s'écouler hors du réservoir, et la valve (11) comprenant des moyens de connexion étanches au tuyau d'injection (5) du dispositif de remplissage selon l'une quelconque des revendications précédentes.

11. Cigarette électronique (10) selon la revendication 9 dans laquelle les moyens de connexion étanches comprennent un connecteur de type Luer femelle ou mâle.

12. Cigarette électronique (10) selon la revendication 10 dans lequel le connecteur de type Luer est du type Luer Lock.

13. Cigarette électronique (10) selon l'une des revendications 9 à 11 dans laquelle la valve unidirectionnelle comprend un ressort (15) poussant une pièce mobile de fermeture (13) contre le culot (16) de la valve (11), la pression appliquée par le ressort (15) entre la pièce mobile de fermeture (13) et le culot (16) assurant l'étanchéité du réservoir de la cigarette électronique (10).
